(19) Europäisches Patentamt

European Patent Office

Office européen des brevets

(11) **EP 0 495 836 B2**

(12) **NEW EUROPEAN PATENT SPECIFICATION**

(45) Date of publication and mention
of the opposition decision:
**20.09.2000 Bulletin 2000/38**

(45) Mention of the grant of the patent:
**17.03.1993 Bulletin 1993/11**

(21) Application number: **90915097.1**

(22) Date of filing: **12.10.1990**

(51) Int. Cl.[7]: **C11D 3/386**

(86) International application number:
**PCT/DK90/00261**

(87) International publication number:
**WO 91/05839 (02.05.1991 Gazette 1991/10)**

(54) **DYE TRANSFER INHIBITION**

VERHINDERUNG DER FARBSTOFFÜBERTRAGUNG

INHIBITION DE TRANSFERT DE COLORANT

(84) Designated Contracting States:
**AT BE CH DE DK ES FR GB GR IT LI LU NL SE**

(30) Priority: **13.10.1989 US 421414**

(43) Date of publication of application:
**29.07.1992 Bulletin 1992/31**

(73) Proprietors:
• **NOVO NORDISK A/S**
**2880 Bagsvaerd (DK)**
• **THE PROCTER & GAMBLE COMPANY**
**Cincinnati, Ohio 45202 (US)**

(72) Inventors:
• **DAMHUS, Ture**
**DK-2100 Copenhagen O (DK)**
• **KIRK, Ole**
**DK-2200 Copenhagen N (DK)**
• **PEDERSEN, Gitte**
**DK-1910 Frederiksberg C (DK)**

• **VENEGAS, Manuel, Garcia**
**Cincinnati, OH 45231 (US)**

(56) References cited:
**EP-A- 173 378          EP-B- 0 072 098**
**WO-A-87/07639          WO-A-89/09813**
**DE-A- 2 009 721        DE-A- 2 430 699**
**GB-A- 2 101 167        SE-B- 358 654**
**US-A- 4 077 768        US-A- 4 690 895**

• **Bumpus,J.A et al,**
**Appl.Environ.Microbiol.,54,1988, 1143-1150**
• **Glenn, J.K. et al,**
**Appl.Environ.Microbiol.,45,1983,1741-1747**
• **Schreiber, W.;Biochem.Biophys.Res.**
**Comm.,63(2), 1975, 509-514**
• **Ben Aziz,A.et al, Phytochemistry,10,1971,1445-1452**
• **Wasserman, B.P.,J.Food Sci.,49,1984,536-538**
• **Dixon,M.et al,"Enzymes",Longmans,1966,183**

EP 0 495 836 B2

## Description

### FIELD OF INVENTION

The present invention relates to an enzymatic process for inhibiting the transfer of dye from a dyed fabric to another fabric during washing, to a bleaching agent for use in the process, and to a process for bleaching dyes in solution.

### BACKGROUND OF THE INVENTION

The use of bleaching agents in washing procedures and as constituents of detergent compositions is well known in the art. Thus, bleaching agents are incorporated in or sold as constituents of a major part of the commercially available detergent compositions. Important conventional bleaching agents incorporated in detergent compositions are compounds which act as precursors of hydrogen peroxide formed in the course of the washing procedure. Perborates and percarbonates are the most important examples of compounds which are employed as bleaching agents and which exert a bleaching effect in this fashion. The detailed mechanism of bleaching by means of these bleaching agents is not known at present, but it is generally assumed that the hydrogen peroxide formed during washing converts coloured substances (responsible for stains on fabric) into non-coloured materials by oxidation and that some oxidation of the coloured substances may also take place due to their direct interaction with perborate or percarbonate.

One drawback of these commonly used bleaching agents is that they are not particularly efficient at the lower temperatures at which coloured fabrics are usually washed. Their efficiency may be enhanced by the use of activators (e.g. organic acid anhydrides, esters or imides) which give rise to the formation of peracids.

Apart from being employed for bleaching stains on fabric, such conventional bleaching agents have also been suggested for preventing surplus dyes from coloured fabrics which leach from the fabrics when these are washed from being deposited on other fabrics present in the same wash (this phenomenon is commonly known as dye transfer). The problem of dye transfer, of course, is most noticeable when white or light-coloured fabrics are washed together with fabrics of a darker colour from which dye is leached during washing.

It has, however, been found that the currently employed bleaching agents, whether activated or not, are not particularly effective in inhibiting dye transfer, possibly because the rate at which they oxidize dissolved dyes is rather slow. On the other hand, peracids formed from the bleaching activators are active against dyes on fabric so as to cause discolouration of the fabric in question.

US 4,077,768 discloses the use of iron porphin, haemin chloride or iron phthalocyanine, or derivatives thereof together with hydrogen peroxide for dye transfer inhibition. It is indicated that these compounds act as catalysts for the bleaching process whereby they provide an increase in the rate at which dissolved dyes are oxidised (or, in other words, bleached) without causing any discolouration of the dye in the fabric. However, these catalysts are destroyed by the presence of excess hydrogen peroxide which makes it necessary to control the release of hydrogen peroxide so that only the quantity of hydrogen peroxide needed to effect the inhibition of dye transfer should be present in the wash water at any time. Such controlled release of the bleaching agent may be difficult to achieve.

Bumpus, J.A., et al., Appl. Environ. Microbiol. 54, 1988, pp. 1143-1150, and Glenn, J.K. et al., Appl. Environ. Microbiol., 45, 1983, pp. 1741-1747, disclose the bleaching of dyes such as Crystal Violet and certain polymeric dyes, respectively, by *Phanerochaete chrysosporum*-derived peroxidase in combination with $H_2O_2$.

### SUMMARY OF THE INVENTION

It has surprisingly been found possible to bleach coloured substances leached from dyed textiles or from textiles soiled with a colourant in a solution of wash liquor thereby preventing the coloured substance in question from being deposited on other textiles in the wash liquor, when enzymes utilizing hydrogen peroxide or molecular oxygen for the oxidation of organic or inorganic substances, including coloured substances, are added to the wash liquor. Such enzymes are usually termed peroxidases and oxidases, respectively.

Accordingly, the present invention relates to a process for inhibiting the transfer of a textile dye from a dyed fabric to another fabric when said fabrics are washed and/or rinsed together in a wash liquor, characterized by comprising:

1)

a) adding an enzyme exhibiting peroxidase activity to the wash liquor in which said fabrics are washed and/or rinsed, and
b) adding hydrogen peroxide, a hydrogen peroxide precursor, or an enzymatic system capable of generating hydrogen peroxide at the beginning of or during the washing and/or rinsing process,

or

2) adding an enzyme exhibiting oxidase activity on phenolic compounds.

In the present context, the term "enzyme exhibiting peroxidase activity" is understood to indicate an enzyme with a mode of action similar to that of a peroxidase and will be used synonymously therewith. Similarly, the term "enzyme exhibiting oxidase activity" is understood to indicate an enzyme with a similar mode of action to that of an oxidase and is meant to be synonymous therewith in the following. Suitable oxidases are those which act on aromatic compounds such as phenols and related substances.

One or more substrates for the enzyme is added at the beginning of or during the washing and/or rinsing process, when the enzyme is one with peroxidase activity. In the case of oxidases, molecular oxygen is usually present in sufficient quantities.

It is well recognized in the art (cf. for instance B.C. Saunders et al., Peroxidase, London, 1964, p. 10 ff.) that peroxidases act on various amino and phenolic compounds resulting in the production of a colour. In view of this, it must be considered surprising that peroxidases (and certain oxidases) may also exert an effect on coloured substances in solution such that dye transfer is inhibited. While the mechanism governing the ability of these enzymes to effect dye transfer inhibition has not yet been elucidated, it is currently believed that the enzymes act by reducing hydrogen peroxide or molecular oxygen and oxidizing the coloured substance (donor substrate) dissolved or dispersed in the wash liquor, thereby either generating a colourless substance or providing a substance which is not adsorbed to the fabric. This reaction is shown in Reaction Scheme 1 below (for peroxidases) and Reaction Scheme 2 below (for oxidases useful for the present purpose)

Reaction Scheme 1:

$$\text{Donor substrate} + H_2O_2 \rightarrow \text{oxidized donor} + 2\ H_2O$$

Reaction Scheme 2:

$$\text{Donor substrate} + O_2 \rightarrow \text{oxidized donor} + 2\ H_2O$$

It has previously been reported that peroxidases may decolourize certain pigments (cf. for instance W. Schreiber, Biochem. Biophys. Res. Commun. 63 (2), 1975, pp. 509-514, describing the degradation of 3-hydroxyflavone by horseradish peroxidase; A. Ben Aziz, Phytochemistry 10, 1971, pp. 1445-1452, describing the bleaching of carotene by means of a peroxidase; and B.P. Wasserman, J. Food Sci. 49, 1984, pp. 536-538, describing the decolourization of betalain by horseradish peroxidase). Ben Aziz et al. and Wasserman et al. present the bleaching action of peroxidases on carotene and betalain, respectively, as a problem when using these pigments as food colourants, which problem must be counteracted by including an antioxidant in the foodstuff in question. Thus, they do not consider the peroxidase-mediated bleaching of these pigments to have any practical utility in itself.

Although these publications describe test methods whereby the respective pigments are incubated with the enzyme in solution, the pigments in question are all pure compounds of natural origin and are also readily bleached by the bleaching agents usually incorporated in modern detergents (cf. for instance Second World Conference on Detergents, A.R. Baldwin (ed.), American Oil Chemist's Society, 1978, pp. 177-180).

Contrary to this, the commonly used textile dyes, when dissolved or dispersed in wash liquors, are generally resistant to oxidation by atmospheric oxygen and also, to a greater or lesser extent, to the bleaching agents currently used in detergents which, as noted in US 4,077,768, are inefficient dye transfer inhibitors as they act too slowly on the dispersed or dissolved dyes. Under these circumstances, it must be considered surprising that the enzymes used in the present process are, in fact, able to oxidize these dyes. Other commonly used bleaching agents which may have an effect on textile dyes in solution or dispersion, e.g. hypochlorite, also attack dye on or in the fabrics, resulting in discolouration thereof. It is an important advantage of the enzymes used in the process of the invention that they do not cause any appreciable colour degradation in the dyed fabric itself. A comprehensive catalogue of commonly used textile dyes, both synthetic (such as azo dyes) and natural or nature-identical (by which is meant a substance which is produced synthetically, but which in structure and properties is identical to the natural compound), e.g. indigo, is found in the Color Index, 3rd ed. Vol. 1-8.

In another aspect, the present invention relates to process for bleaching textile dyes in solution or dispersion, characterized by comprising adding to said solution or dispersion an enzyme exhibiting oxidase activity on phenolic compounds, and an additional oxidisable substrate.

It is contemplated that, apart from having utility in inhibiting dye transfer during a washing or rinsing process, the ability of these enzymes to bleach dyes in solution may also make them useful for treating waste water from the textile industry forming part of a waste disposal process.

In a further aspect, the present invention relates to a bleaching agent for inhibiting the transfer of a textile dye from a dyed fabric to another fabric when said fabrics are washed and/or rinsed together in a wash liquor, characterized by comprising an enzyme exhibiting oxidase activity on phenolic compounds, in the form of a non-dusting granulate, a stabilized liquid or a protected enzyme.

Apart from this utility, the bleaching agent may also be employed in the treatment of waste water from the textile and possibly also other industries, as indicated above.

## DETAILED DISCLOSURE OF THE INVENTION

Examples of suitable oxidases which act on aromatic compounds, in particular phenolic, e.g. polyphenolic, are catechol oxidase (EC 1.10.3.1) or laccase (EC 1.10.3.2). For the sake of convenience, such oxidases, and peroxidases are collectively termed bleaching enzymes in the following.

Bleaching enzymes which may be employed for the present purpose may be isolated from and are producible by plants (e.g. horseradish peroxidase) or microorganisms such as fungi or bacteria. Some preferred fungi include strains belonging to the subdivision Deuteromycotina, class Hyphomycetes, e.g. *Fusarium, Humicola, Tricoderma, Myrothecium, Verticillum, Arthromyces, Caldariomyces, Ulocladium, Embellisia, Cladosporium* or *Dreschlera*, in particular *Fusarium oxysporum* (DSM 2672), *Humicola insolens, Trichoderma reseei, Myrothecium verrucana* (IFO 6113), *Verticillum alboatrum, Verticillum dahlie, Arthromyces ramosus* (FERM P-7754), *Caldariomyces fumago, Ulocladium chartarum, Embellisia allior Dreschlera halodes*.

Other preferred fungi include strains belonging to the subdivision Basidiomycotina, class Basidiomycetes, e.g. *Coprinus, Phanerochaete, Coriolus* or *Trametes*, in particular *Coprinus cinereus* f. *microsporus* (IFO 8371), *Phanerochaete chrysosporum,* (NA-12), *Coprinus macrorhizus,* or *Coriolus versicolor* (e.g. PR4 28-A).

Further preferred fungi include strains belonging to the subdivision Zygomycotina, class Mycoraceae, e.g. *Rhizopus* or *Mucor*, in particular *Mucor hiemalis*.

Some preferred bacteria include strains of the order Actinomycetales, e.g. *Streptomyces spheroides* (ATTC 23965), *Streptomyces thermoviolaceus* (IFO 12382) or *Streptoverticillum verticillium* ssp. *verticillium*

Other preferred bacteria include *Bacillus pumillus* (ATCC 12905), *Bacillus stearothermophilus, Rhodobacter sphaeroides, Rhodomonas palustri, Streptococcus lactis, Pseudomonas purrocinia* (ATCC 15958) or *Pseudomonas fluorescens* (NRRL B-11).

Other potential sources of useful bleaching enzymes (in particular peroxidases) are listed in B.C. Saunders et al., op. cit., pp. 41-43.

Methods of producing enzymes to be used according to the invention are described in the art, cf. for example FEBS Letters 1625, 173(1), Applied and Environmental Microbiology, Feb. 1985, pp. 273-278, Applied Microbiol. Biotechnol. 26, 1987, pp. 158-163, Biotechnology Letters 9(5), 1987, pp. 357-360, Nature 326, 2 April 1987, FEBS Letters 4270, 209(2), p. 321, EP 179 486, EP 200 565, GB 2 167 421, EP 171 074, and Agric. Biol. Chem. 50(1), 1986, p. 247.

Particularly preferred bleaching enzymes are those which are active at the typical pH of washing liquors, i.e. at a pH of 6.5 - 10.5, preferably 6.5 - 9.5, and most preferably 7.5 - 9.5. Such enzymes may be isolated by screening for the relevant enzyme production by alkalophilic microorganisms, e.g. using the ABTS assay described in R.E. Childs and W.G. Bardsley, Biochem. J. 145, 1975, pp. 93-103.

Other preferred bleaching enzymes are those which exhibit a good thermostability as well as a good stability towards commonly used detergent components such as non-ionic, cationic, or anionic surfactants, detergent builders, phosphate etc.

Another group of useful bleaching enzymes are haloperoxidases, such as chloro- and bromoperoxidases.

The bleaching enzyme may furthermore be one which is producible by a method comprising cultivating a host cell transformed with a recombinant DNA vector which carries a DNA sequence encoding said enzyme as well as DNA sequences encoding functions permitting the expression of the DNA sequence encoding the enzyme, in a culture medium under conditions permitting the expression of the enzyme and recovering the enzyme from the culture.

A DNA fragment encoding the enzyme may, for instance, be isolated by establishing a cDNA or genomic library of a microorganism producing the enzyme of interest, such as one of the organisms mentioned above, and screening for positive clones by conventional procedures such as by hybridization to oligonucleotide probes synthesized on the basis of the full or partial amino acid sequence of the enzyme, or by selecting for clones expressing the appropriate enzyme activity, or by selecting for clones producing a protein which is reactive with an antibody against the native enzyme.

Once selected, the DNA sequence may be inserted into a suitable replicable expression vector comprising appropriate promotor, operator and terminator sequences permitting the enzyme to be expressed in a particular host organism, as well as an origin of replication enabling the vector to replicate in the host organism in question.

The resulting expression vector may then be transformed into a suitable host cell, such as a fungal cell, preferred examples of which are a species of *Aspergillus*, most preferably *Aspergillus oryzae* or *Aspergillus niger*. Fungal cells may be transformed by a process involving protoplast formation and transformation of the protoplasts followed by regeneration of the cell wall in a manner known per se. The use of *Aspergillus* as a host microorganism is described in EP 238,023 (of Novo Industri A/S), the contents of which are hereby incorporated by reference.

Alternatively, the host organisms may be a bacterium, in particular strains of *Streptomyces* and *Bacillus*, or *E. coli*. The transformation of bacterial cells may be performed according to conventional methods, e.g. as described in T. Maniatis et al., Molecular Cloning: A Laboratory Manual, Cold Spring Harbor, 1982.

The screening of appropriate DNA sequences and construction of vectors may also be carried out by standard procedures, cf. T. Maniatis et al., op. cit.

The medium used to cultivate the transformed host cells may be any conventional medium suitable for growing the host cells in question. The expressed enzyme may conveniently be secreted into the culture medium and may be recovered therefrom by well-known procedures including separating the cells from the medium by centrifugation or filtration, precipitating proteinaceous components of the medium by means of a salt such as ammonium sulphate, followed by chromatographic procedures such as ion exchange chromatography, affinity chromatography, or the like.

When the bleaching enzyme employed in the invention is a peroxidase, $H_2O_2$ may be added at the beginning or during the process, e.g. in an amount of 0.001-5 mM, particularly 0.01-1 mM. When using *Coprinus* peroxidase, 0.01-0.25 mM $H_2O_2$ is preferred, and with *B. pumilus* peroxidase 0.1-1 mM $H_2O_2$.

When the bleaching enzyme employed in the process of the invention is a peroxidase, it may be desirable to utilize an enzymatic process for hydrogen peroxide formation. Thus, the process according to the invention may additionally comprise adding an enzymatic system (i.e. an enzyme and a substrate therefor) which is capable of generating hydrogen peroxide at the beginning or during the washing and/or rinsing process.

One such category of hydrogen peroxide generating systems comprises enzymes which are able to convert molecular oxygen and an organic or inorganic substrate into hydrogen peroxide and the oxidized substrate, respectively. These enzymes produce only low levels of hydrogen peroxide, but they may be employed to great advantage in the process of the invention as the presence of peroxidase ensures an efficient utilization of the hydrogen peroxide produced.

Preferred hydrogen peroxide-generating enzymes are those which act on cheap and readily available substrates which may conveniently be included into detergent compositions. An example of such a substrate is glucose which may be utilized for hydrogen peroxide production by means of glucose oxidase. Other suitable oxidases are urate oxidase, galactose oxidase, alcohol oxidases, amine oxidases, amino acid oxidase and cholesterol oxidase.

It has surprisingly been found that the addition of another oxidisable substrate (for the bleaching enzyme used in the process of the invention) at the beginning or during the washing and/or rinsing process may enhance the dye transfer inhibitory effect of the bleaching enzyme employed. This is thought to be ascribable to the formation of short-lived radicals or other oxidised states of this substrate which participate in the bleaching or other modification of the coloured substance. Examples of such oxidisable substrates are metal ions, e.g. $Mn^{++}$, halide ions, e.g. chloride or bromide ions, or organic compounds such as phenols, e.g. p-hydroxycinnamic acid or 2,4-dichlorophenol. Other examples of phenolic compounds which may be used for the present purpose are those given in M. Kato and S. Shimizu, Plant Cell Physiol. 26(7), 1985, pp. 1291-1301 (cf. Table 1 in particular) or B.C. Saunders et al., op. cit., p. 141 ff. The amount of oxidisable substrate to be added is suitably between about 1µM and 1mM.

In the process of the invention, the bleaching enzyme will typically be added as a component of a detergent composition. As such, it may be included in the detergent composition in the form of a non-dusting granulate, a liquid, in particular a stabilized liquid, or a protected enzyme. Non-dusting granulates may be produced, e.g., as disclosed in US 4,106,991 and 4,661,452 (both to Novo Industri A/S) and may optionally be coated by methods known in the art. Liquid enzyme preparations may, for instance, be stabilized by adding a polyol such as propylene glycol, a sugar or sugar alcohol, lactic acid or boric acid according to established methods. Other enzyme stabilizers are well known in the art. Protected enzymes may be prepared according to the method disclosed in EP 238,216. The detergent composition may also comprise one or more substrates for the enzyme.

The detergent composition will additionally comprise surfactants which may be of the anionic, non-ionic, cationic, amphoteric, or zwitterionic type as well as mixtures of these surfactant classes. Typical examples of anionic surfactants are linear alkyl benzene sulfonates (LAS), alpha olefin sulfonates (AOS), alcohol ethoxy sulfates (AES) and alkali metal salts of natural fatty acids.

The detergent composition may further contain other detergent ingredients known in the art as e.g. builders, anti-corrosion agents, sequestering agents, anti-soil redeposition agents, perfumes, enzyme stabilizers, etc.

It is at present contemplated that, in the process of the invention, the bleaching enzyme may be added in an amount of 0.01-100 mg enzyme per liter of wash liquor.

The detergent composition may be formulated in any convenient form, e.g. as a powder or liquid. The enzyme may be stabilized in a liquid detergent by inclusion of enzyme stabilizers as indicated above. Liquid detergents may further include stabilized hydrogen peroxide precursors. Usually, the pH of a solution of the detergent composition of the invention will be 7-12 and in some instances 7.0-10.5. Other detergent enzymes such as proteases, lipases or amylases may be included in the detergent composition.

## EXAMPLES

Dyes were purchased from Aldrich Chemicals. Peroxycarboxylic acid references were synthesized according to W.E. Parker, C. Ricciuti, C.L. Ogg and D. Swern, J. Am. Chem. Soc., **77**, 4037 (1955). Spectra were recorded on a Hewlett Packard 8451 diode array spectrophotometer. The samples were scanned over the wavelength range 200 to 800 nm for one minute (spectra recorded every 6 sec). **CMP** is used below as abbreviation for peroxidase derived form *Coprinus macrorhizus* (obtained from Chemical Dynamics). $H_2O_2$ is used synonymously with hydrogen peroxide. 2,4-DCP and PCA are used as abbreviations of 2,4-dichlorophenol and p-coumaric acid.

## EXAMPLE 1

Bleaching of Congo Red in solution

To a solution of Congo Red (0.058 mM, 42 mg/l (dye content 93 %, giving an initial absorbance at 486 nm of 2.0) in phosphate buffer pH 7 (0.1 M) was added as bleaching agent either 2 mM $H_2O_2$, 1 mM peroxyoctanoic acid, or 2.5 mg/l **CMP** + 0.25 mM $H_2O_2$. The experiments were performed at 25 °C in 1 cm quartz cells containing 1 ml. As listed below, only the peroxidase system gave any bleaching effect (monitored as observed change in absorbance at 486 nm in one minute).

| Bleaching system | Delta absorbance in 1 min |
|---|---|
| 2 mM $H_2O_2$ | 0.00 |
| 1 mM Peroxyoctanoic acid | 0.00 |
| 2.5 mg/l **CMP** + 0.25 mM $H_2O_2$ | 0.18 |

## EXAMPLE 2

Bleach acceleration by phenolic compounds

Experiments were performed according to example 1, except that the accelerating effect of adding various phenolic compounds as an additional substrate along with the peroxidase and $H_2O_2$ was examined. 2,4-DCP and PCA were added at a level of only 5 μM (0.82 mg/l in both cases).

| Bleaching system | Δ absorbance in 1 min |
|---|---|
| 2.5 mg/l **CMP** + 0.25 mM $H_2O_2$ | 0.18 |
| 2.5 mg/l **CMP** + 0.25 mM $H_2O_2$ + 5 μM 2,4-DCP | 0.74 |
| 2.5 mg/l **CMP** + 0.25 mM $H_2O_2$ + 5 μM PCA | 0.28 |

## EXAMPLE 3

Bleaching of Acid Blue 45 in solution

Experiments were performed according to example 1 only using a solution of Acid Blue 45 (0.058 mM, 68 mg/l (dye content ca 40 %), giving an initial absorbance at 594 nm of 1.0). Bleaching was measured as change in absorbance at 594 nm.

| Bleaching system | Δ absorbance in 1 min |
|---|---|
| 2 mM $H_2O_2$ | 0.00 |
| 1 mM Peroxyoctanoic acid | 0.00 |
| 2.5 mg/l **CMP** + 0.25 mM $H_2O_2$ | 0.42 |

## EXAMPLE 4

Bleach acceleration by phenolic compounds

Experiments were performed as described in example 2 except for using Acid Blue 45 as described in example 3.

| Bleaching system | Δ absorbance in 1 min |
|---|---|
| 2.5 mg/l **CMP** + 0.25 mM $H_2O_2$ | 0.42 |
| 2.5 mg/l **CMP** + 0.25 mM $H_2O_2$ + 5 μM 2,4-DCP | 0.69 |
| 2.5 mg/l **CMP** + 0.25 mM $H_2O_2$ + 5 μM PCA | 0.98 |

## EXAMPLE 5

Solutions of Congo Red and Acid Blue 45 prepared, according to example 1 and 3, were treated with laccase (100 mg/l, crude enzyme preparation, derived from *Myceliophthora thermophile,* available from Novo Nordisk as a special preparation, SP 315. Further information is available upon request). The difference in absorbance relative to a solution without enzyme added was measured after an incubation time of 16 hours.

| Bleaching agent | Δ in absorbance after 16 hr. | |
|---|---|---|
| | Congo Red (486 nm) | Acid Blue 45 (594 nm) |
| 0.1 g/l laccase | 0.29 | 0.09 |

## EXAMPLE 6

Dye adsorption to textiles

In order to demonstrate that the effects seen in the above solution experiments are reflected on textiles present in such solutions, experiments were carried out in which clean cotton swatches were immersed in solutions of model textile dyes.

In one such experiment, the clean swatches were immersed in 0.058 mM and 0.012 mM solutions, respectively, of the dye Acid Blue 45 in 50 mM phosphate buffer (pH 7.0, 25°C) and agitated for 60 min. The phosphate buffer was freshly prepared from water of a hardness equivalent to 1.6 mM $Ca^{2+}$. The swatch load was approx. 11 g cotton cloth/l.

Afterwards the swatches were rinsed in tap water and air-dried in the dark on a clean towel overnight. The the remission at 600 nm (absorption region for blue substances) was measured on a Datacolor Elrephometer 2000.

The results of three treatments within the above prescriptions were as follows:

| Remission at 600 nm (%) | | |
|---|---|---|
| | Swatches retrieved from 0.058 mM Acid Blue 45 solution | Swatches retrieved from 0.012 mM Acid Blue 45 solution |
| 1. Reference (buffer only) | 60 | 80 |
| 2. 0.2 mM $H_2O_2$ | 58 | 79 |
| 3. $H_2O_2$ as in 2 + 20 mg/l **CMP** | 74 | 90 |

Higher remission numbers here correspond to less blue color. Thus, the dye deposition on the clean swatches is considerably less in the solutions with peroxidase present.

## EXAMPLE 7

Dye adsorption to textiles

In another experiment, the procedure of example 6 was repeated in every detail, except that the dye in the solutions was Congo Red (at the same mM levels). Here, visual inspection of the resulting swatches unequivocally demonstrated the effect of the peroxidase: treatments 1 and 2 gave indistinguishably and heavily red-colored swatches, whereas only a faint yellowish color was seen on the swatches from treatment 3.

## EXAMPLE 8

Dye adsorption to textiles

In this experiment, a particular type of test swatch was added for demonstrating dye adsorption effects. Each swatch consisted of 6 strips of textile, each 1.5 cm by 5 cm, sown together; the 6 textile brands were triacetate, bleached cotton, nylon, polyester, orlon, and viscose rayon.

The model washing liquor was a phosphate buffer prepared as in example 6 with 0.6 g/l linear alkylbenzenesulfonate added as a surfactant. Two 7 cm by 7 cm clean cotton swatches and one of the above multiswatches (also clean) were immersed in l litre of the washing liquor, with Congo Red added to a level of 0.012 mM, in each of two Terg-o-tometer beakers. In beaker 1, the bleaching system consisted of $H_2O_2$ at a level of 2 mM, in beaker 2, 20 mg of **CMP** was further added. A wash of 30 min at 40°C with 60 rotations/min was performed, after which the swatches were rinsed in tap water and dried as above (example 6). This time, Hunter color difference readings were obtained for the multiswatches as follows:

| Hunter color difference readings | | |
|---|---|---|
| | Beaker 1 (only $H_2O_2$) | Beaker 2 ($H_2O_2$ + CMP) |
| Triacetate | 7.5 | 2.0 |
| Cotton | 69.9 | 35.0 |
| Nylon | 57.2 | 23.4 |
| Polyester | 16.0 | 5.0 |
| Orlon | 27.4 | 9.8 |
| Viscose | 69.7 | 30.7 |
| (A value of 0 here indicates no change in color from the clean swatch and increasing numbers correspond to a visual impression of deeper color.) | | |

Thus, the conclusion from example 6 is also valid here for all the textile brands studied.

### EXAMPLE 9

Dye transfer from textile to textile

Swatches dyed with Congo Red as a model dye for azo textile dyes was prepared by immersing clean cotton swatches in a bath of Congo Red and sodium sulfate in demineralized water and keeping them there during a gradual heating to 90°C, ending with addition of further sodium sulfate and a period of a constant temperature of 90°C. After being dyed the swatches were rinsed in cold tap water and dried overnight between layers of gauze.

In the present experiment, washing was carried out in three Terg-o-tometer beakers under the same general conditions as in example 8. The contents of the beakers were:

Beaker 1: Only phosphate buffer with LAS (as in example 8)
Beaker 2: Buffer + LAS + 2 mM $H_2O_2$
Beaker 3: As 2 with 20 mg/l **CMP** added

In each beaker was introduced 2 Congo Red swatches, 7 cm by 7 cm, and one clean multiswatch (see example 8). After washing and drying as in example 8, the Hunter readings of the multiswatches were as follows:

|  | Beaker 1 | Beaker 2 | Beaker 3 |
|---|---|---|---|
| Triacetate | 3.4 | 3.4 | 2.8 |
| Cotton | 45.7 | 45.3 | 36.6 |
| Nylon | 41.6 | 40.9 | 35.6 |
| Polyester | 7.9 | 7.4 | 6.7 |
| Orlon | 14.7 | 15.0 | 11.2 |
| Viscose | 45.1 | 44.6 | 36.3 |

Thus, the swatches in beaker 1 suffer a substantial dye transfer which is not remedied by hydrogen peroxide alone, but reduced significantly by the peroxidase treatment.

The red swatches from the three beakers had essentially identical readings, showing that the peroxidase treatment does not change the dyeing any more than the other treatments.

### EXAMPLE 10

Dye adsorption to textiles

For the purpose of studying the peroxidase effect in a more realistic washing environment, a powder detergent was composed as follows:

| Component | w/w % active material |
|---|---|
| Sodium carbonate | 22 |
| Sodium diphosphate | 17 |
| Sodium silicate | 7 |
| Sodium triphosphate | 5 |
| Sodium perborate monohydrate | 4 |
| Sodium nonanoyloxybenzenesulfonate | 5 |

(continued)

| Component | w/w % active material |
|---|---|
| Sodium linear alkylbenzenesulfonate | 9 |
| Sodium alkyl sulfate | 4 |
| Various minor components: alcohol ethoxylate, diethylenetriamine pentaacetate, polyacrylate, polyethylene glycol, protease, optical brightener: each < 1 Sodium sulfate and other miscellaneous: balance. | |

This detergent was used at a level of 2 g/l in water of a hardness equivalent to 1 .6 mM $Ca^{2+}$ to produce a washing liquor in which pH was adjusted to 8.5. In this washing liquor, Congo Red was dissolved to a level of 0.012 mM. Beaker 1 was the reference (detergent + Congo Red); in beaker 2, **CMP** was added to a level of 20 mg/l. In both beakers, two clean cotton swatches and one clean multiswatch were added as in example 8. All other conditions were as in example 8 and the Hunter data for the multiswatches after the wash were as follows:

| | Beaker 1 | Beaker 2 |
|---|---|---|
| Triacetate | 4.0 | 1.1 |
| Cotton | 62.5 | 2.3 |
| Nylon | 48.0 | 1.1 |
| Polyester | 4.0 | 0.4 |
| Orlon | 18.4 | 1.2 |
| Viscose | 66.3 | 1.3 |

Once again, the peroxidase clearly reduces - here almost eliminates - the amount of color deposited on the swatches.

## EXAMPLE 11

Dye transfer from textile to textile

In this example, the detergent solution from example 10 was used in a Terg-O-Tometer trial where two of the Congo Red-dyed swatches described above (example 9) were washed together with one clean multiswatch in each of two beakers. Beaker 1 contained just 1 litre of detergent solution, beaker 2 additionally contained 20 mg/l of **CMP**. The remaining conditions were as in example 8. The swatches, after retrieval from the washing liquor, rinsing and drying as above, showed the following Hunter color difference data:

| | Beaker 1 | Beaker 2 |
|---|---|---|
| Triacetate | 2.3 | 1.1 |
| Cotton | 47.0 | 13.1 |
| Nylon | 36.0 | 11.3 |
| Polyester | 2.1 | 1.1 |
| Orlon | 6.5 | 2.7 |
| Viscose | 48.7 | 10.6 |

A considerable transfer of dye was thus observed in beaker 1, and this was significantly reduced by adding the peroxidase to the washing liquor.

Again, the dyed swatches were checked also, and no color difference was seen between the two treatments.

## EXAMPLE 12

Bleaching of dyestuffs in solution

Peroxidase activity: In this example, the peroxidase activity is measured as follows. The following are mixed in a 30°C thermostated 1 ml quartz cuvette:

200 µl 1 mM 4-aminoantipyrine (Sigma No. A-4382, 0.2 mg/ml)
200 µl N-ethyl-N-sulphobutyl-m-toluidin-Na (ESBT, 5.86 mg/ml)
200 µl 0.5 M phosphate buffer, pH 7.0
200 µl enzyme sample, diluted to 0.02-0.10 NOPA/ml

200 µl 10 mM hydrogen peroxide is added, and the absorbance at 550 nm is followed for 1 minute. The activity (in NOPA units) is calculated as the increase in absorbance within the first minute after addition of $H_2O_2$ multiplied by the dilution. The enzyme sample should be diluted so that the increase in absorbance per minute is within the limits 0.02 to 0.10.

Peroxidase production from *Bacillus pumilus*: Media were prepared as follows (ingredients in g/l):

|  | TY*3 |
|---|---|
| Trypticase, BBL g/l | 60 |
| Yeast Extract, Difco g/l | 15 |
| FeSO4*7H2O g/l | 0.025 |
| MnSO4*4H2O g/l | 0.0026 |
| MgSO4*7H2O g/l | 0.045 |
| pH 7.3 (Adjusted with KOH) The medium was autoclaved at 121 °C for 45 minutes | |

|  | Agar3 |
|---|---|
| Pepton Bacto g/l | 6 |
| Pepticase g/l | 4 |
| Yeast Extract, Difco g/l | 3 |
| Meat Extract g/l | 1.5 |
| Glucose | 1 |
| pH | 7.3 |
| Agar (from Merck) | 20 (added last) |
| The agar was autoclaved at 121 °C for 45 minutes | |

Inoculum agar : 10 Agar3 slants were inoculated with a freeze-dried peroxidase-producing strain of *B.*

*pumilus* and incubated at 30°C for 24 hours.

Inoculum media : Two 500 ml Shake flasks containing 100 ml TY*3 media were inoculated with one Agar3 slant and incubated at 30°C and 250 rpm for 24 hours.

Peroxidase production : 50 Shake flasks containing 100 ml of TY*3 were inoculated each with 2 ml of inoculum described above. Then 2.5 ml of a sterile 40% (w/w) Glucose in water was added to each shake flask. The shake flasks were incubated at 30°C for 48 hrs and then harvested. The final peroxidase activity was 1 NOPA/ml.

3250 ml culture broth were filtered through a Seitz Supra 100 filterplate and secondly through a Supra 50 plate to obtain a clear filtrate with an activity of 1.29 NOPA/ml.

Bleaching of dyes in solution: The above clear filtrate from *B. pumilus* (BPP) was tested. The dyestuffs tested were Direct Blue 1 (C.I. #24410, product of Keystone Aniline), Acid Red 151 (C.I. #26900, product of Sandoz), Procion Blue H ERD (product of ICI) and Procion Blue EXL (product of ICI).

A reaction solution was prepared, containing 50 mM sodium phosphate, 0.3 NOPA/ml of peroxidase, dyestuff (as indicated below) corresponding to an absorption maximum (in the visible range) of 0.025-0.035, and 0.25 mM $H_2O_2$ at room temperature at the pH indicated below. After addition of $H_2O_2$ (added last), a spectral scan was made every minute for 12 minutes. Below, the change in absorbance at the wavelength of maximum absorption is listed.

| Dyestuff | pH | Absorbance change immediately/after 12 min | Wavelength |
|---|---|---|---|
| Acid Red 151 | 7.0 | 0.030/0.032 | 513 nm |
| | 9.0 | 0.033/0.033 | 513 - |
| | 10.5 | 0.027/0.030 | 513 - |
| Direct Blue 1 | 7.0 | 0.024/0.025 | 597 nm |
| | 9.0 | 0.022/0.026 | 597 - |
| | 10.5 | 0.009/0.023 | 597 - |
| Procion Blue H ERD | 7.0 | 0.022/0.021 | 617 nm |
| | 9.0 | 0.009/0.022 | 617 - |
| | 10.5 | 0.001/0.010 | 617 - |
| Procion Blue H EXL | 9.0 | 0.021/0.026 | 626 nm |
| | 10.5 | 0.016/0.025 | 626 - |

When the two values of the absorbance change are close, the bleaching is practically instantaneous. Generally, bleaching over the entire visible range follows the above trends at the absorbance maximum.

In all cases, use of 0.25 mM $H_2O_2$ without enzyme left the dye unchanged.

**Claims**

**1.** A process for inhibiting the transfer of a textile dye from a dyed fabric to another fabric when said fabrics are washed and/or rinsed together in a wash liquor, characterized by comprising:

1)

a) adding an enzyme exhibiting peroxidase activity to the wash liquor in which said fabrics are washed and/or rinsed, and
b) adding hydrogen peroxide, a hydrogen peroxide precursor, or an enzymatic system capable of generating hydrogen peroxide at the beginning of or during the washing and/or rinsing process,

or
2) adding an enzyme exhibiting oxidase activity on phenolic compounds.

**2.** A process according to Claim 1, wherein the peroxidase is derived from a strain of *Coprinus* or *B. pumilus*.

3. A process according to any preceding claim, wherein the hydrogen peroxide precursor is a perborate or percarbonate.

4. A process according to Claim 1, wherein the enzyme exhibiting oxidase activity is catechol oxidase (EC 1.10.3.1) or laccase (EC 1.10.3.2).

5. A process according to any preceding claim, wherein the textile dye is a synthetic dye such as an azo dye, or a natural or nature-identical dye such as indigo.

6. A process according to any preceding claim, wherein an additional oxidisable substrate such as a metal ion, a halide ion or an organic compound such as a phenol. e.g. p-hydroxycinnamic acid or 2,4-dichlorophenol, is added at the beginning of or during the washing and/or rinsing process.

7. A process according to Claim 6, wherein the amount of oxidisable substrate added is between 1 $\mu$M and 1 mM.

8. A process according to any preceding claim, wherein the enzyme is one producible by a microorganism, particularly a bacterium or fungus, e.g. an actinomycete or basidiomycete.

9. A process according to any preceding claim, wherein the enzyme is of plant origin.

10. A process according to any preceding claim, wherein the enzyme is one producible by a method comprising cultivating a host cell transformed with a recombinant DNA vector carrying a DNA sequence encoding said enzyme as well as DNA sequences encoding functions permitting the expression of the enzyme, in a culture medium under conditions permitting the expression of the enzyme and recovering the enzyme from the culture.

11. A process according to any of Claims 1, 3 and 5 - 10, wherein the enzyme exhibiting peroxidase activity is a haloperoxidase, such as a chloro or bromo peroxidase.

12. A process according to any preceding claim, wherein the enzyme is active at a pH of 6.5-10.5, preferably 6.5-9.5, and most preferably 7.5-9.5.

13. A process according to any preceding claim, wherein the enzyme is added in an amount of 0.01-100 mg/l of wash liquor.

14. A process according-to any preceding claim, wherein the enzyme is incorporated in a detergent composition.

15. A process for bleaching textile dyes in solution or dispersion, characterized by comprising adding to said solution or dispersion an enzyme exhibiting oxidase activity on phenolic compounds, and an additional oxidisable substrate for said enzyme.

16. A process according to Claim 15, wherein the enzyme exhibiting oxidase activity is catechol oxidase (EC 1.10.3.1) or laccase (EC 1.10.3.2).

17. A process according to Claim 15 or 16, wherein the enzyme is active at a pH of 6.5-10.5, preferably 6.5-9.5, and most preferably 7.5-9.5.

18. A process according to any of Claims 15 - 17, wherein the textile dye is a synthetic dye such as an azo dye, or a natural or nature-identical dye such as indigo.

19. A process according to any of Claims 15 - 18, wherein the additional oxidisable substrate is a metal ion, a halide ion or an organic compound such as a phenol. e.g. p-hydroxycinnamic acid or 2,4-dichlorophenol.

20. A process according to any of claims 15-29, wherein the amount of oxidisable substrate added is between 1 $\mu$M and 1mM.

21. A bleaching agent for inhibiting the transfer of a textile dye from a dyed fabric to another fabric when said fabrics are washed and/or rinsed together in a wash liquor, characterized by comprising an enzyme exhibiting oxidase activity on phenolic compounds, in the form of a non-dusting granulate, a stabilized liquid or a protected enzyme.

22. A bleaching agent according to Claim 21, wherein the enzyme exhibiting oxidase activity is catechol oxidase (EC 1.10.3.1) or laccase (EC 1.10.3.2).

**Patentansprüche**

1. Verfahren zur Hemmung der Übertragung eines Textilfarbstoffes von einem gefärbten Gewebe auf ein anderes Gewebe, wenn besagte Gewebe zusammen in einer Waschlauge gewaschen und/oder gespült werden, dadurch gekennzeichnet, daß es umfaßt:

   1)

   a) Zugeben eines Enzyms, das Peroxidaseaktivität zeigt, zur Waschlauge, in der besagte Gewebe gewaschen und/oder gespült werden, und
   b) Zugeben von Wasserstoffperoxid, einem Wasserstoffperoxid-Vorläufer oder einem enzymatischen System, das in der Lage ist, Wasserstoffperoxid zu erzeugen, am Beginn oder während des Wasch- und/oder Spülprozesses,

   oder
   2) Zugeben eines Enzyms, das Oxidaseaktivität auf Phenolverbindungen zeigt.

2. Verfahren nach Anspruch 1, dadurch gekennzeichnet, daß die Peroxidase aus einem Stamm von Coprinus oder B. pumilus gewonnen ist.

3. Verfahren nach Anspruch 1 oder 2, dadurch gekennzeichnet, daß der Wasserstoffperoxid-Vorläufer ein Perborat oder Percarbonat ist.

4. Verfahren nach Anspruch 1, dadurch gekennzeichnet, daß das Enzym, das Oxidaseaktivität zeigt, Catechol-Oxidase (EC 1.10.3.1) oder Laccase (EC 1.10.3.2) ist.

5. Verfahren nach einem der vorangehenden Ansprüche, dadurch gekennzeichnet, daß der Textilfarbstoff ein synthetischer Farbstoff, wie etwa ein Azo-Farbstoff, oder ein natürlicher oder naturidentischer Farbstoff, wie etwa Indigo, ist.

6. Verfahren nach einem der vorangehenden Ansprüche, dadurch gekennzeichnet, daß ein zusätzliches oxidierbares Substrat, wie etwa ein Metallion, ein Halogenidion oder eine organische Verbindung, wie etwa Phenol, z.B. p-Hydroxyzimtsäure oder 2,4-Dichlorphenol, am Beginn oder während des Wasch- und/oder Spülprozesses zugesetzt wird.

7. Verfahren nach Anspruch 6, dadurch gekennzeichnet, daß die Menge an zugesetztem oxidierbaren Substrat zwischen 1 μM und 1 mM liegt.

8. Verfahren nach einem der vorangehenden Ansprüche, dadurch gekennzeichnet, daß das Enzym eines ist, das von einem Mikroorganismus produzierbar ist, insbesondere von einem Bakterium oder einem Pilz, z.B. einem Actinomyceten oder Basidiomyceten.

9. Verfahren nach einem der vorangehenden Ansprüche, dadurch gekennzeichnet, daß das Enzym pflanzlichen Ursprungs ist.

10. Verfahren nach einem der vorangehenden Ansprüche, dadurch gekennzeichnet, daß das Enzym eines ist, das mit einer Methode herstellbar ist, die umfaßt, daß eine Wirtszelle, die mit einem rekombinanten DNA-Vektor transformiert ist, der eine DNA-Sequenz, die besagtes Enzym kodiert, sowie DNA-Sequenzen, die Funktionen kodieren, die die Expression des Enzyms erlauben, trägt, in einem Kulturmedium unter Bedingungen kultiviert wird, die die Expression des Enzyms erlauben, und daß das Enzym aus der Kultur gewonnen wird.

11. Verfahren nach einem der Ansprüche 1, 3 und 5-10, dadurch gekennzeichnet, daß das Enzym, das Peroxidaseaktivität zeigt, eine Haloperoxidase, wie etwa eine Chlor- oder Bromperoxidase, ist.

12. Verfahren nach einem der vorangehenden Ansprüche, dadurch gekennzeichnet, daß das Enzym bei einem pH von 6,5-10,5, vorzugsweise 6,5-9,5 und am bevorzugtesten 7,5-9,5 aktiv ist.

13. Verfahren nach einem der vorangehenden Ansprüche, dadurch gekennzeichnet, daß das Enzym in einer Menge von 0,01-100 mg/l Waschlauge zugegeben wird.

14. Verfahren nach einem der vorangehenden Ansprüche, dadurch gekennzeichnet, daß das Enzym in eine Waschmittelzusammensetzung eingemischt wird.

15. Verfahren zum Bleichen von Textilfarbstoffen in Lösung oder Dispersion, dadurch gekennzeichnet, daß es umfaßt, daß zu besagter Lösung oder Dispersion ein Enzym, das Oxidaseaktivität auf Phenolverbindungen zeigt, und ein zusätzliches oxidierbares Substrat für das Enzym zugegeben werden.

16. Verfahren nach Anspruch 15, dadurch gekennzeichnet, daß das Enzym, das Oxidaseaktivität zeigt, Catechol-Oxidase (EC 1.10.3.1) oder Laccase (EC 1.10.3.2) ist.

17. Verfahren nach Anspruch 15 oder 16, dadurch gekennzeichnet, daß das Enzym bei einem pH von 6,5-10,5, vorzugsweise 6,5-9,5 und am bevorzugtesten 7,5-9,5, aktiv ist.

18. Verfahren nach einem der Ansprüche 15 bis 17, dadurch gekennzeichnet, daß der Textilfarbstoff ein synthetischer Farbstoff, wie etwa ein Azo-Farbstoff, oder ein natürlicher oder naturidentischer Farbstoff, wie etwa Indigo, ist.

19. Verfahren nach einem der Ansprüche 15 bis 18, dadurch gekennzeichnet, daß das zusätzliche oxidierbare Substrat ein Metallion, ein Halogenidion oder eine organische Verbindung ist, wie etwa Phenol, z.B. p-Hydroxyzimtsäure oder 2,4-Dichlorphenol.

20. Verfahren nach einem der Ansprüche 15 bis 19, dadurch gekennzeichnet, daß die Menge an zugegebenem oxidierbaren Substrat zwischen 1 µM und 1 mM liegt.

21. Bleichmittel zur Hemmung der Übertragung eines Textilfarbstoffes von einem gefärbten Gewebe auf ein anderes Gewebe, wenn besagte Gewebe zusammen in einer Waschlauge gewaschen und/oder gespült werden, dadurch gekennzeichnet, daß es ein Enzym, das Oxidaseaktivität auf Phenolverbindungen zeigt, in Form eines nichtstaubenden Granulats, einer stabilisierten Flüssigkeit oder eines geschützten Enzyms umfaßt.

22. Bleichmittel nach Anspruch 21, dadurch gekennzeichnet, daß das Enzym, das Oxidaseaktivität zeigt, Catechol-Oxidase (EC 1.10.3.1) oder Laccase (EC 1.10.3.2) ist.

**Revendications**

1. Procédé pour inhiber le transfert d'un colorant pour textiles d'un tissu teint à un autre tissu quand lesdits tissus sont lavés et/ou rincés ensemble dans une liqueur de lavage, caractérisé en ce qu'il comprend:

    1)

        a) l'addition d'une enzyme manifestant une activité de peroxydase à la liqueur de lavage dans laquelle lesdits tissus sont lavés et/ou rincés, et
        b) l'addition de peroxyde d'hydrogène, d'un précurseur de peroxyde d'hydrogène ou d'un système enzymatique capable de donner naissance à du peroxyde d'hydrogène, au début ou au cours de l'opération de lavage et/ou de rinçage,

    ou bien
    2) l'addition d'une enzyme manifestant une activité d'oxydase sur des composés phénoliques.

2. Procédé selon la revendication 1, dans lequel la peroxydase est derivée d'une souche de *Coprinus* ou de *B. pumilus*.

3. Procédé selon la revendication 1 ou 2, dans lequel le précurseur de peroxyde d'hydrogène est un perborate ou un percarbonate.

EP 0 495 836 B2

**4.** Procédé selon la revendication 1, dans lequel l'enzyme manifestant une activité d'oxydase est la catécholoxydase (EC 1.10.3.1) ou la laccase (EC 1.10.3.2).

**5.** Procédé selon l'une quelconque des revendications précédentes, dans lequel le colorant pour textiles est un colorant synthétique tel qu'un colorant azoïque, ou un colorant naturel ou identique à la nature tel que l'indigo.

**6.** Procédé selon l'une quelconque des revendications précédentes, dans lequel on ajoute au début ou au cours de l'opération de lavage et/ou de rinçage un substrat oxydable supplémentaire tel qu'un ion métallique, un ion halogénure ou un composé organique tel qu'un phénol, par exemple l'acide p-hydroxycinnamique ou le 2,4-dichlorophénol.

**7.** Procédé selon la revendication 6, dans lequel la quantité de substrat oxydable ajouté est comprise entre 1 µM et 1 mM.

**8.** Procédé selon l'une quelconque des revendications précédentes, dans lequel l'enzyme est une enzyme pouvant être produite par un microorganisme, en particulier par une bactérie ou un champignon, par exemple un actinomycète ou un basidiomycète.

**9.** Procédé selon l'une quelconque des revendications précédentes, dans lequel l'enzyme est d'origine végétale.

**10.** Procédé selon l'une quelconque des revendications précédentes, dans lequel l'enzyme est une enzyme pouvant être produite par une méthode comprenant la culture d'une cellule hôte transformée avec un vecteur d'ADN recombiné portant une séquence d'ADN codant pour ladite enzyme ainsi que des séquences d'ADN codant pour des fonctions permettant l'expression de l'enzyme, dans un milieu de culture dans des conditions permettant l'expression de l'enzyme, et la récupération de l'enzyme à partir de la culture.

**11.** Procédé selon l'une quelconque des revendications 1, 3 et 5-10, dans lequel l'enzyme manifestant une activité de peroxydase est une haloperoxydase telle qu'une chloro- ou une bromoperoxydase.

**12.** Procédé selon l'une quelconque des revendications précédentes, dans lequel l'enzyme est active à un pH de 6,5-10,5, de préférence de 6,5-9,5, et de façon la plus préférable de 7,5-9,5.

**13.** Procédé selon l'une quelconque des revendications précédentes, dans lequel l'enzyme est ajoutée en une quantité de 0,01-100 mg/l de liqueur de lavage.

**14.** Procédé selon l'une quelconque des revendications précédentes, dans lequel l'enzyme est incorporée dans une composition détergente.

**15.** Procédé pour décolorer des colorants pour textiles en solution ou en dispersion, caractérisé en ce qu'il comprend l'addition à ladite solution ou dispersion d'une enzyme manifestant une activité d'oxydase sur des composés phénoliques et un substrat oxydable supplémentaire pour l'enzyme.

**16.** Procédé selon la revendication 15, dans lequel l'enzyme manifestant une activité d'oxydase est la catécholoxydase (EC 1.10.3.1) ou la laccase (EC 1.10.3.2).

**17.** Procédé selon la revendication 15 ou 16, dans lequel l'enzyme est active à un pH de 6,5-10,5, de préférence de 6,5-9,5, de façon la plus préférable de 7,5-9,5.

**18.** Procédé selon l'une quelconque des revendications 15-17, dans lequel le colorant pour textiles est un colorant synthétique tel qu'un colorant azoïque, ou un colorant naturel ou identique à la nature tel que l'indigo.

**19.** Procédé selon l'une quelconque des revendications 15-18, dans lequel le substrat oxydable supplémentaire est un ion métallique, un ion halogénure ou un composé organique tel qu'un phenol, par exemple l'acide p-hydroxycinnamique ou le 2,4-dichlorophénol.

**20.** Procédé selon l'une quelconque des revendications 15-19, dans lequel la quantité de substrat oxydable ajouté est comprise entre 1 µM et 1 mM.

16

21. Agent décolorant pour inhiber le transfert d'un colorant pour textiles d'un tissu teint à un autre tissu quand lesdits tissus sont lavés et/ou rincés ensemble dans une liqueur de lavage, caractérisé en ce qu'il comprend une enzyme manifestant une activité d'oxydase sur des composés phénoliques, sous forme d'un produit granulé ne formant pas de poussière, d'un liquide stabilisé ou d'une enzyme protégée.

22. Agent décolorant selon la revendication 21, dans lequel l'enzyme manifestant une activité d'oxydase est la catécholoxydase (EC 1.10.3.1) ou la laccase (EC 1.10.3.2).